# EUROPEAN PATENT SPECIFICATION

(11) **EP 2 412 795 B1**
(45) Date of publication and mention of the grant of the patent: **28.12.2016**
(21) Application number: 10755867.8
(22) Date of filing: 10.03.2010
(51) Int. Cl.: A61L 27/36, A61L 27/26, A61L 27/38, A61L 27/60, C12M 1/12, C12N 11/02

(54) **CROSSLINKED MATERIAL COMPRISING ELASTIN AND COLLAGEN, AND USE THEREOF**
VERNETZTES MATERIAL MIT ELASTIN UND COLLAGEN SOWIE VERWENDUNG
MATÉRIAU RÉTICULÉ À BASE D'ÉLASTINE ET DE COLLAGÈNE, ET SON UTILISATION

(30) Priority: 27.03.2009 JP 2009079898; 13.11.2009 JP 2009260000
(43) Date of publication of application: 01.02.2012
(73) Proprietor: Maruha Nichiro Corporation, Tokyo 135-8608 (JP); Sapporo Medical University, Hokkaido 060-0061 (JP); Hirosaki University, Hirosaki-shi, Aomori 036-8560 (JP)
(72) Inventor: MATSUMOTO, Yoshitaka, Sapporo-shi Hokkaido 060-8556 (JP); YOTSUYANAGI, Takatoshi, Sapporo-shi Hokkaido 060-8556 (JP); SUTO, Shinichi, Hirosaki-shi Aomori 036-8560 (JP); YAMAYA, Yoshifumi, Tsukuba-shi Ibaraki 300-4295 (JP); HOSHINO, Yosuke, Tsukuba-shi Ibaraki 300-4295 (JP); NISHIMURA, Kazunari, Tokyo 135-8608 (JP); KOGA, Tomoko, Tsukuba-shi Ibaraki 300-4295 (JP); ENARI, Hiroyuki, Tsukuba-shi Ibaraki 300-4295 (JP)
(74) Representative: Banfi, Paolo
(86) International application number: PCT/JP2010/054001
(87) International publication number: WO 2010/110067

(56) References cited:
- EP-A1- 0 602 297
- EP-A1- 1 283 063
- EP-A1- 1 642 599
- WO-A1-02/096978
- WO-A2-2007/126411
- US-A1- 2004 265 785
- US-B1- 6 660 280
- FRIESS W ED - LEHR CLAUS-MICHAEL DAUM NICOLE SCHNEIDER MARC SCHAFER ULRICH F: "Collagen - biomaterial for drug deliveryDedicated to Professor Dr. Eberhard Nu@?rnberg, Friedrich-Alexander-Universita@?t Erlangen-Nu@?rnberg, on the occasion of his 70th birthday", EUROPEAN JOURNAL OF PHARMACEUTICS AND BIOPHARMACEUTICS, ELSEVIER SCIENCE PUBLISHERS B.V., AMSTERDAM, NL, vol. 45, no. 2, 1 March 1998 (1998-03-01), pages 113-136, XP004256962, ISSN: 0939-6411, DOI: 10.1016/S0939-6411(98)00017-4
- AYAKO HONMURA ET AL.: 'Synergistic effect of collagen peptide and elastin peptide derived from salmon on promoting hyaluronic acid production and cell growth of normal human dermal fibroblast cells (SF-TY)' BULLETIN OF THE JAPANESE SOCIETY OF SCIENTIFIC FISHERIES vol. 75, no. 1, 15 January 2009, pages 86 - 88, XP008154599
- MOTOHIKO TADA ET AL.: 'Sake Yurai Elestin Collagen Peptide no Hifu ni Taisuru Koka' JAPAN SOCIETY FOR BIOSCIENCE, BIOTECHNOLOGY, AND AGROCHEMISTRY 05 March 2008, page 281, XP008162677
- TAKATOSHI YOTSUYANAGI ET AL.: 'Sake Yurai Collagen /Elastin Takotai no Tokusei to Igakuteki Oyo' INOBESHON JAPAN 2009 KOSHIKI GAIDO BUKKU 16 September 2009, page 47, XP008154679

## Description

### Technical Field

The present invention relates to a crosslinked material using a fish-derived elastin and a fish-derived collagen, and use thereof.

### Background Art

Vulnerary covering materials or artificial dermis using collagen are disclosed in the specifications of, for example, Japanese Patent Nos. 2997823, 3772232, and 3105308, and some of them are commercially available. Most of the collagen used as such materials is collected from the tissue of domestic animals such as cows and pigs. Recently, however, problems of BSE (bovine spongiform encephalopathy) have been elicited, and it is feared that humans may be infected with pathogens from collagen products using mammalian-derived raw materials including bovine hide.

Under such circumstances, development of artificial dermis using materials other than materials derived from mammalian such as cows and pigs has been desired with the following requirements:
- It has high bio-compatibility.
- It is excellent in invasiveness into cells.
- It does not cause suppression of wound contraction, and rapidly shows degradability in the living body after the formation of dermis-like tissue. A foam sheet containing a fish dermis collagen and use thereof is disclosed in Japanese Patent Laid-Open No. 2005-95331.

By the way, collagen is denatured into gelatin when it is heated in an aqueous solution or suspension. Since the denatured collagen is easily dissolved in water and has a low strength, it is degraded by a proteolytic enzyme in the living body and easily absorbed in the living body. Therefore it becomes unsuitable for medical materials that require appropriate stability in the living body. In addition, a fish-derived collagen has disadvantages that the denaturation temperature is low and the gel strength is weak as compared with a mammalian-derived collagen. In order to prevent denaturation, a reaction is required to be carried out at temperature that is not higher than the denaturation temperature, but the reaction does not proceed at low temperatures depending upon the types of crosslinking agents. It is a defect of the fish-derived collagen.

On the other hand, elastin is a main protein of the elastic fiber in various tissues and organs such as ligament and skin, and use of elastin as medical materials for regenerating damaged tissues or organs has been considered.

Japanese Patent Publication No. 30616/1994 discloses a molding composition produced by mixing a water-soluble elastin and a water-soluble collagen while cooling. WO2002/96978 discloses a crosslinked elastin produced by chemically bonding a component such as collagen to elastin, while elastin is crosslinked with a crosslinking agent, as well as an instrument for medical use thereof and regenerated tissue. US 6,660,280 describes a collagen product containing collagen of marine origin preferably from fish skin. The collagen is crosslinked, formed into the product and dried by lyophilization. The product can be used e.g. as a cell culture support, for cartilage reconstruction or as a healing dressing.

### Reference List

### Patent Literature

Patent Literature 1: Japanese Patent No. 2997823
Patent Literature 2: Japanese Patent No. 3772232
Patent Literature 3: Japanese Patent No. 3105308
Patent Literature 4: Japanese Patent Laid-Open No. 2005-95331
Patent Literature 5: Japanese Patent Publication No. 30616/1994
Patent Literature 6: WO2002/96978
Patent Literature 7: US 6,660,280

### Summary of Invention

### Technical Problems to be Solved by the Invention

Attention has been paid to collagen and elastin as materials for producing artificial dermis or medical materials for regenerating tissue, but in the present state, materials that satisfy origins of raw materials, handling property and mechanical strength of the resultant products, properties corresponding to the intended use have not been found yet.

For example, no literatures have disclosed vulnerary covering materials or artificial dermis using a fish-derived collagen and a fish-derived water soluble elastin as materials having origins other than cows and pigs. In addition, the fish-derived collagen has disadvantages that the denaturation temperature is low and the gel strength is weak as compared with a mammalian-derived. These points are defect of such materials. In order to prevent denaturation, a reaction is required to be carried out at temperature that is not higher than the denaturation temperature, but the reaction does not proceed at such a low temperature depending upon the types of crosslinking agents.

Japanese Patent Publication No. 30616/1994 discloses a molding composition produced by mixing a water-soluble elastin and a water-soluble collagen while cooling and a membrane-like structural material obtained by using the same. However, since the structure obtained from the molding composition is not treated to make it insoluble by such as crosslinking, it cannot be maintained in its own form in water for a long time in some cases. Furthermore, the artificial dermis is a porous material, but it is not easy to obtain a porous material as an artificial dermis from the molding composition described in this publication.

WO2002/96978 discloses a crosslinked product, in which collagen is chemically bonded when a water soluble elastin is crosslinked with a crosslinking agent, and use thereof as a medical material. However, WO2002/96978 discloses that it is difficult not only to pour an elastincontaining material into a mold, but also to control the reaction rate depending upon crosslinking agents or crosslinking conditions, and that a crosslinked product having a desired shape and properties. Furthermore, even if a crosslinked product can be obtained by the crosslinking method, the obtained product cannot sufficiently be used for various uses such as medical materials including artificial dermis or scaffold materials for cell culture in some cases.

An object of the present invention is to provide a collagen/elastin crosslinked material that is fully applicable to various uses such as medical materials including artificial dermis and scaffolding materials for cell culture.

### Means for Solving the Problems

A porous crosslinked material of the present invention is characterized in that it is obtained by crosslinking a fish-derived collagen and a fish-derived elastin to each other, through a production method comprising the steps of:
- preparing a solution in which a fish-derived collagen and
   a fish-derived elastin having a weight-average molecular weight of 10,000 to 100,000 are dissolved at a temperature of 0 to 10°C and pH ranging from 3 to 6, whereby coacervation of the elastin is not generated;
- obtaining a dried product from the solution; and
- forming a crosslinked material by crosslinking the dried product.

The method of producing a crosslinked material according to the present invention is characterized in that a water-insoluble crosslinked material can be obtained by crosslinking with a crosslinking agent, crosslinking by heating, crosslinking by irradiation of an ultraviolet ray or a radioactive ray, or a combination of one or more of these crosslinking processes, as a method of crosslinking the dry material which is not crosslinked yet.

A medical material of the present invention is characterized in comprising the above-mentioned crosslinked material. Furthermore, a scaffolding material for cell culture of the present invention is characterized in comprising the above-mentioned crosslinked material. Furthermore, a cosmetics base material of the present invention is characterized in comprising the above-mentioned crosslinked material.

### Effects of the Invention

The present invention can provide a collagen/elastin crosslinked material obtained from fish-derived raw materials, which is satisfactorily applicable to various uses, for example, medical materials including artificial dermis and scaffolding materials for cell culture. The present invention can provide a method of producing the collagen/elastin crosslinked material. The present invention can further provide various uses of the collagen/elastin crosslinked material as a medical material, a scaffolding material for cell culture and a base material for cosmetics.

### Brief Description of Drawings

[Figure 1] Figure 1 is a graph showing a result of a cytotoxic test of a fish-derived atelocollagen in Example 1 (mitomycin C (cytotoxic substance, commercially available from Kyowa Hakko Kirin Co., Ltd.)).
[Figure 2] Figure 2 is a graph showing a result of a cytotoxic test of a fish-derived water soluble elastin in Example 1 (mitomycin C (cytotoxic substance, commercially available from Kyowa Hakko Kirin Co., Ltd.)).
[Figure 3] Figure 3 is a scanning electron microscope image (SEM image: 200-times magnification) of a fish-derived collagen/elastin porous material using a crosslinking agent in Example 2.
[Figure 4] Figure 4 is a scanning electron microscope image (SEM image: 200-times magnification) of a fish-derived collagen/elastin porous material using thermal crosslinking in Example 3.
[Figure 5] Figure 5 is a graph showing a breaking strength of a porous material with the addition of elastin to collagen in Example 4.
[Figure 6] Figure 6 is a graph showing a breaking distance of a porous material with the addition of elastin to collagen in Example 4.
[Figure 7] Figure 7 is a tissue image (macro image) of a collagen/elastin porous material on Day 56 of subcutaneous implantation thereof into the rat in Example 6.
[Figure 8] Figure 8 is a graph showing the change of an area of wound in Example 7 (the area immediately after operation is defined as 100%).
[Figure 9] Figure 9 is a picture showing a state of a wound in a sample group (on Day 14 after operation) in Example 7.
[Figure 10] Figure 10 is a scanning electron microscope image (SEM image: 100-times magnification) of a DNA-added collagen/elastin porous material using a crosslinking agent in Example 8.
[Figure 11] Figure 11 is a scanning electron microscope image (SEM image: 100-times magnification) of a protamine-added collagen/elastin porous material using a crosslinking agent in Example 9.
[Figure 12] Figure 12 is a scanning electron microscope image (SEM image: 100-times magnification) of a hydroxyapatite-added collagen/elastin porous material using a crosslinking agent in Example 10.
[Figure 13] Figure 13 is a picture showing a result in which a tissue cultured by using a collagen/elastin porous material as a scaffolding material for cell culture was stained with alcian blue in Example 11.
[Figure 14] Figure 14 is a picture showing a result in which a tissue cultured by using a collagen/elastin porous material as a scaffolding material for cell culture was stained with toluidine blue in Example 11.
[Figure 15] Figure 15 is a picture showing a result in which a tissue cultured by using a collagen/elastin porous material as a scaffolding material for cell culture was immunostained with a collagen II antibody in Example 11.
[Figure 16] Figure 16 is a photograph image of a film-like collagen/elastin complex (water retaining state) in Example 12.
[Figure 17] Figure 17 is a graph showing a measurement result of skin viscoelasticity in Example 13. (n = 4, comparison with CES + skin lotion applied portion, t-test, **p < 0.01, *p < 0.05)
[Figure 18] Figure 18 is a photograph image of a film-like collagen/elastin complex (water retaining state) in Example 14.

### Description of Embodiments

In the present invention, a fish-derived elastin is used as elastin. Fishes used for preparing elastin includes those in the family *Salmonidae* of the order *Salmoniformes* such as salmon and trout; those in the family *Scombridae* of the order *Perciformes* such as tuna and bonito; those in the family *Carangidae* of the order *Perciformes* such as yellowtail, those in the family *Hexagrammidae* of the order *Scorpaeniformes* such as Ling Cod. As elastin, a water-soluble elastin is preferable, in order to obtain a homogeneous crosslinked material in the formation of a crosslinked material with collagen. Examples of the water soluble elastin include tropoelastin that is a biosyntheses precursor of elastin; α-elastin; and κ-elastin obtained by treating elastin with acid and alkali; as well as a hydrolysate(s) of elastin obtained by treating elastin with an enzyme. Desirable elastin to be used in the present invention is α-elastin which is a high molecular weight material composed of molecules having a uniform molecular weight, because a mechanical strength can be easily obtained in the crosslinked material thus prepared.

The molecular weight of elastin to be used in the present invention is about 10,000 to 100,000.

As compared with collagen, elastin is degraded in a living body slower, but elastin has high bio-compatibility similar to collagen. The mechanical strength and flexibility are expected to be added by the addition of elastin. Furthermore, the α-elastin is said to have biological functions such as a macrophage migrating activity, cell adhesion, inhibition of platelet aggregation, and when the α-elastin is used as a raw material for the crosslinked material, such biological functions are expected to be added.

Preparation of elastin from a fish can be carried out by a conventional method(s).

Elastin derived from fishes contains a smaller amount of desmosine and isodesmosine as one type of amino acid involved in the crosslinked structure in an elastin molecule as compared with elastin from cows and pigs. The fish-derived elastin is characterized in that its amino acid composition of is different from elastin from mammalian such as cows and pigs.

The following composition (mol%) of amino acids of a fish-derived elastin, which can be used in the present invention, in which the contents of the amino acids are different from those of elastin derived from animals, are described below as an example.
- glycine: 35 to 45%
- alanine: 10 to 20%
- valine: 3 to 8%
- isodesmosine: 0.1 to 1.0%
- desmosine: 0.1 to 1.0%
- total of nonpolar amino acid: 67 to 77%

The amino acids as the objects of the analysis in the above-mentioned amino acid composition include glycine (Gly), alanine (Ala), valine (Val), leucine (Leu), isoleucine (Ile), serine (Ser), threonine (Thr), aspartic acid (Asp), glutamic acid (Glu), lysine (Lys), arginine (Arg), histidine (His), hydroxylysine (Hylys), cysteine (Cys), methionine (Met), tyrosine (Tyr), phenyl alanine (Phe), proline (Pro), hydroxyproline (Hypro), isodesmosine (Iso-Des), and desmosine (Des). The contents (mol%) of the amino acids other than glycine, alanine, valine, isodesmosine, and desmosine are described below as one example:
- leucine: 3.4 to 4.5
- isoleucine: 0.9 to 1.9
- serine: 3.1 to 3.4
- threonine: 3.1 to 6.5
- aspartic acid: 1.6 to 2.9
- glutamic acid: 3.2 to 4.3
- lysine: 0.7 to 1.5
- arginine: 1.6 to 2.6
- histidine: 0.2 to 0.8
- hydroxylysine: 0
- cysteine: 0 to 0.2
- methionine: 0.4 to 0.7
- tyrosine: 3.4 to 4.4
- phenyl alanine: 1.1 to 3.1
- proline: 8.6 to 10.1
- hydroxyproline: 0.5 to 0.9

As collagen, a fish-derived collagen is used. Fishes used for preparing collagen includes those in the family *Salmonidae* of the order *Salmoniformes* such as salmon and trout, those in the family *Scombridae* of the order *Perciformes* such as tuna and bonito, those in the family *Carangidae* of the order *Perciformes* such as yellowtail, those in the family *Gadidae* of the order *Gadiformes* such as Alaska cod and Alaska Pollack. As collagen, a soluble (water soluble) collagen is preferable, in order to obtain a homogeneous crosslinked material in the formation of a crosslinked material with elastin. Preparation of collagen from fish can be carried out by a conventional method(s). Telopeptide that is a main antigen site of collagen is present on both ends of collagen. When this part is removed by enzymatic treatment, the antigenicity of collagen is extremely lowered. Such collagen is called as atelocollagen, and is applied for medical implant materials or scaffolding materials for tissue engineering. As the soluble collagen, which can be used in the present invention, a fish-derived atelocollagen is more preferable.

Furthermore, the molecular weight of collagen, which can be used in the present invention, is not particularly limited, but the weight-average molecular weight is preferably about 10,000 to 300,000.

A fish-derived collagen has a different amino acid composition from that of collagen of cows and pigs in that the fish-derived collagen contains a small content of proline and hydroxyproline. Furthermore, collagen has a characteristic that it is denatured at a denaturation temperature or a temperature higher than the denaturation temperature and becomes gelatin. However, the denaturation temperature of the fish-derived collagen is generally lower than that derived from cows and pigs.

The following amino acid composition (mol%) of the fish-derived collagen, is an example, which can be used in the present invention. The following amino acid composition is different from those of collagen derived from animals:
- glycine: 34 to 40%
- proline: 5.0 to 11.0%
- hydroxyproline: 4.0 to 8.0%
- histidine: 0.8 to 2.0%

The amino acids as the objects of the analysis in the above-mentioned amino acid composition include glycine (Gly), alanine (Ala), valine (Val), leucine (Leu), isoleucine (Ile), serine (Ser), threonine (Thr), aspartic acid (Asp), glutamic acid (Glu), lysine (Lys), arginine (Arg), histidine (His), hydroxylysine (Hylys), cysteine (Cys), methionine (Met), tryptophane (Trp), tyrosine (Tyr), phenyl alanine (Phe), proline (Pro), and hydroxyproline (Hypro).

The contents (mol%) of the amino acids other than the above-mentioned glycine, proline, hydroxyproline, and histidine are described below as one example:
- alanine: 10.5 to 15.0
- valine: 1.1 to 2.2
- leucine: 1.5 to 2.3
- isoleucine: 0.5 to 1.1
- serine: 3.5 to 6.3
- threonine: 2.1 to 3.6
- aspartic acid: 4.3 to 5.3
- glutamic acid: 7.4 to 9.5
- lysine: 2.4 to 2.9
- arginine: 4.9 to 5.5
- hydroxylysine: 0.5 to 0.8
- cysteine: 0 to 0.2
- methionine: 0.3 to 1.7
- tryptophane: 0
- tyrosine: 0.2 to 0.3
- phenyl alanine: 1.1 to 1.4

A crosslinking agent may be used to form a crosslinked material of elastin and collagen. In particular, when strength stability is required for a crosslinked material requires, a crosslinking agent is preferably used. Any crosslinking agents may be used as long as they can crosslink elastin and collagen. Examples thereof may include carbodiimide-based, azide-based, fluorophosphate-based, triazine-based, epoxy-based crosslinking agents (condensing agents). Furthermore, a condensing auxiliary agent may be used. Examples thereof may include N-hydroxypolycarboxylic imides, N-hydroxytriazoles, triazines, and ethyl ester of 2-hydroxyimino-2-cyanoacetic acid. Regarding these condensing agents and condensing auxiliary agents as assistants for condensation, when they are used as medical material, agents having a low residual property and toxicity are desirably selected.

A crosslinked material of the present invention can be produced by crosslinking a fish-derived elastin and a fish-derived collagen by a crosslinking method with the use of a crosslinking agent, a crosslinking method with heat, a crosslinking method with ultraviolet rays or a crosslinking method with radioactive rays. In order to obtain properties for medical materials including artificial dermis, scaffolding materials for cell culture and base materials for cosmetics, a crosslinked material is preferably produced by a production method including the following steps:
(A) a step of preparing a solution in which a fish-derived collagen and a fish-derived elastin having a weight-average molecular weight of 10,000 to 100,000 are dissolved at a temperature of 0 to 10°C and pH ranging from 3 to 6, whereby coacervation of elastin is not generated.
(B) a step of obtaining a crosslinked material from the solution obtained in the step (A) in the condition that coacervation of elastin is not generated.

As the step (B), the following step (B-1) or (B-2) can be used:
(B-1) a step of obtaining a crosslinked material by forming crosslink during lyophilization by adding a crosslinking agent into the solution obtained in the step (A) in a condition that coacervation of elastin is not generated.
(B-2) a step of crosslinking a dried material obtained by drying the solution obtained in the step (A) in the condition that coacervation of elastin is not generated.

As a drying method in the step (B-2), lyophilization, vacuum drying, airblowing drying, drying with a dehydrating solvent, natural drying can be used. Furthermore, following the step (B), crosslinking with the use of a crosslinking agent, crosslinking with heat, crosslinking with ultraviolet rays or radioactive rays may be further added singly or in combination two or more thereof.

Therefore, the production method is characterized in a step of dissolving elastin in a condition that coacervation of elastin is not generated, and, in this state, adding a crosslinking agent into the solution in which elastin and collagen are dissolved and forming crosslink structures during lyophilization to obtain a crosslinked material; or a step of drying the solution in a condition that coacervation of elastin is not generated and crosslinking the dried material. There are such conditions including temperature and pH that a phenomenon so called coacervation is allowed to occur in an aqueous solution. When the coacervation occurs, the water soluble elastin is aggregated on the bottom of the liquid in a shape of starch syrup, and is not crosslinked uniformly with other mixture. In addition, the water soluble elastin is not utterly crosslinked depending upon the types of the crosslinking agent and crosslinking conditions, and the water-soluble elastin is separated from other mixture. Therefore, it is preferable that coacervation of elastin be not allowed to occur in a solution for crosslinking. By reacting elastin with collagen and a crosslinking agent in a condition that coacervation of elastin is not generated, a uniform reaction of elastin with collagen and a crosslinking agent proceeds. As a result, a homogeneous crosslinked material can be obtained. Also when a crosslinked material is obtained as a porous material, a porous material having a homogeneous distribution of pores can be formed. When crosslinking is carried out in a state in which coacervation of elastin is generated, a portion in which elastin is unevenly distributed in a crosslinked material is generated, and a case in which a crosslinked structure is not sufficiently formed on the entire crosslinked material may occur.

The conditions in which coacervation does not occur can be set according to the types of the water soluble elastin to be used.

As the other property, collagen is denatured into gelatin at a certain temperature or higher. Since the denatured collagen is easily dissolved in water, but it has a low strength, it is degraded by a proteolytic enzyme in the living body and easily absorbed in the living body. Therefore, the denatured collagen becomes unsuitable for medical materials to which an appropriate stability in the living body is required. For the reasons above, it is necessary to carry out the preparation of the solution and the crosslinking reaction at a denaturation temperature or lower. In general, the denaturation temperature of collagen derived from fish is said to be about 20°C, and therefore it is desirable that the preparation of the solution and the crosslinking reaction are carried out in the temperature range lower than the above. Furthermore, collagen has characteristics that it is solubilized in a region of neutral region from acid region, but it is insolubilized in alkaline region. Thus, in order to cross-link a composition containing collagen in a uniform condition, the collagen solution has pH of from 1 to 7, and further preferably has pH of from 3 to 6.

Therefore, in order to obtain a homogeneous solution of elastin and collagen, it is necessary that both elastin and collagen are dissolved. More specifically, the temperature is 10°C or less than 10°C at which elastin does not cause coacervation and collagen is not denatured, and further preferably is 0°C to 5°C, and pH is 3 to 6 in which elastin does not cause coacervation and collagen is dissolved. In addition, a pH condition is further preferable that the selected crosslinking agent is easily reacted.

Examples of the solvent for dissolving elastin and collagen may include water, or water containing acid, alkali, and/or surfactant, water containing water soluble organic solvent with low concentration.

In the mixing rate (% by weight) of elastin and collagen, it is preferable that elastin is mixed in a mixing rate of 0.5% to 99.5% with respect to the weight of a crosslinked material. More preferably, the rate is 1 to 50%. In this range, an excellent porous molded material can be obtained, and excellent molded material can be obtained as instruments for medical use including artificial dermis, scaffold materials for cell culture and base materials for cosmetics.

As the method of crosslinking the dried material containing elastin and collagen, chemical crosslinking with a crosslinking agent, crosslinking with heat, crosslinking with ultraviolet rays or radioactive rays can be used. More specifically, dried materials containing elastin and collagen can be crosslinked by at least one of heat, ultraviolet rays and radioactive rays without adding a crosslinking agent.

Furthermore, the crosslinking of a dried material can be carried out in a solvent if necessary. For example, the crosslinking can be carried out in a liquid medium in which the dried material is not dissolved, if necessary, in the presence of a crosslinking agent. The liquid media include an aqueous solution of salts with high concentration, in organic solvents such as alcohols, DMSO, DMF, and dioxane, as well as a mixture of two or more thereof,

The blending rate (% by weight) of the crosslinking agent is not particularly limited, but it is preferably 1 to 50% with respect to the weight of composition to be crosslinked. In order to obtain a molded material excellent as instruments for medical use including artificial dermis, scaffold materials for cell culture and base materials for cosmetics, the blending rate is particularly preferable in the range from 1% to 30%.

When elastin and collagen are crosslinked in a solution, these concentrations (% by weight) of elastin and collagen are not particularly limited for their crosslinking. When the dissolving property of collagen and elastin, and the molding property by the viscosity of a mixture solution are taken into consideration, their concentrations are preferably from 0.5% to 50%, respectively. In order to obtain a molded material excellent as instruments for medical use including artificial dermis, scaffold materials for cell culture and base materials for cosmetics, the concentration is particularly preferable in the range from 1% to 10%.

A molding method of a collagen/elastin crosslinked material of the present invention is not particularly limited, but it is possible to use a mold for molding, which is used for molding general synthetic resin. For example, a molded material can be obtained by adding a crosslinking agent into a solution containing elastin and collagen and pouring the mixture solution into a mold, followed by lyophilization thereof, thus crosslinking is carried out. A molded material can be also obtained by pouring a solution containing elastin and collagen into a mold, and drying the mixed solution to obtain a molded dried material having a shape of a sponge, and crosslinking the dried material. The collagen/elastin crosslinked material, a thread, a membrane, a stick, a pellet, or a tube, reflecting a template to be used can be molded in such a manner. Furthermore, the collagen/elastin crosslinked material can be molded in a membrane shape by a solution casting method. For example, a mixture obtained by adding a crosslinking agent into a solution containing elastin and collagen is spread thinly on a base plate such as a resin and a metal, followed by drying and removing a solvent, and thereby a crosslinked product having a film shape is obtained. A collagen/elastin crosslinked material having a shape of film can be also obtained by spreading a solution containing elastin and collagen thinly on a base plate such as a resin, followed by drying and removing a solvent, and crosslinking the dried material.

It is possible to add fish-derived DNA or protamine to the crosslinked material of the present invention in addition to elastin and collagen.

DNA has a hyaluronic acid production promoting effect with respect to human skin normal fibroblast, and deoxyribonucleotides as the degradation product thereof have a proliferation activity of the human skin normal fibroblast or a collagen production promoting effect. As the fish-derived DNA, DNA derived from family *Salmonidae* can be used. Furthermore, the molecular weight of DNA, which can be used in the present invention is not particularly limited, but the weight-average molecular weight is preferably about 10000 to 100000.

Protamine is a protein specifically existing in the sperm nucleus of vertebrate animals and has an antibacterial effect, and therefore it is expected to have an action of preventing the infection of wound. As the protamine, salmine derived from family Salmonidae and clupeine derived from family Clupeidae can be used.

Regarding amino acids constituting protamine, a large amount of arginine is contained. The content of arginine in salmine as protamine derived from family *Salmonidae* is about 70%. When protamine is contained in the collagen/elastin porous material, arginine is generated in the degradation process in a living body. Arginine suppresses the excess expression of cytokine (physiologically active substance that gives stimulus) released in accordance with an inflammation reaction of the tissue. Furthermore, arginine is a representative component producing NO (nitrogen monoxide), and NO has a function of attacking tumor cells or infected cells. Furthermore, arginine plays an important role for maturity of T-lymphocyte. As mentioned here, arginine has an activity of immune reaction, an effect of promoting cell proliferation, a collagen production promoting effect, and is an important amino acid for promoting the curing of wound or bedsore.

Therefore, the above-mentioned effects can be expected by adding such fish-derived DNA or protamine into a collagen/elastin crosslinked material. These components are blended in such a blending amount that the objective physical properties, properties or functions can be provided in a crosslinked material. The blending amount is not particularly limited, but 0.5% to 50%, and preferably 1% to 20% of DNA and/or protamine is added with respect to the total weight of collagen and elastin.

Furthermore, in the crosslinked material of the present invention, in addition to the fish-derived elastin and collagen, powder-state or granulestate calcium phosphate can be added. This crosslinked material can be applied for a bone regeneration inducing material, or a regeneration inducing material, or bone supply materials for inducing or promoting bone formation in a defective part of bone, but the application is not limited thereto.

Calcium phosphate is blended in such a blending amount that the objective physical properties, properties or functions can be provided in a crosslinked material. The blending amount is not particularly limited, but 0.5% to 50%, and preferably 1 % to 20% of calcium phosphate is added with respect to the total weight of collagen and elastin.

Examples of calcium phosphate may include hydroxyapatite (HA), tribasic calcium phosphate (α-TCP, β-TCP), dicalcium phosphate dihydrate (DCPD), octa-calcium phosphate (OCP), tetracalcium phosphate (TeCP), carbonate apatite (CAP) at least one of which can be used.

According to the present invention, a crosslinked material including a water-insoluble and homogeneous complex can be obtained, and when it is formed in a porous material, a porous structure suitable for artificial dermis and scaffolding material for cell culture can be obtained as a crosslinked material. Furthermore, elastin and collagen, which can be used for producing a crosslinked material according to the present invention, are not derived from conventional mammalian that has a fear of infectious diseases such as BSE (bovine spongiform encephalopathy). On the other hand, the fish-derived natural materials are used, and, therefore, it is possible to provide a highly safely crosslinked material.

By adding elastin, the crosslinked material according to the present invention has improved stability and mechanical strength in the living body as compared with a crosslinked material from collagen alone, and has degradation property in a living body, and has also a suitable physical property as a medical material.

The porous material of the crosslinked material according to the present invention is homogeneous, and has a porous structure suitable for artificial dermis or scaffolding material for cell culture. Therefore, when it is used as the artificial dermis, the dermal fibroblasts are excellent in invasiveness and excellent in granulation formation, wound contraction is suppressed. The porous material is absorbed in a living body rapidly after wound is cured. Consequently, when the skin is damaged by wound, heat injury, bedsore, the porous material is applied to the damaged surface, and used for defective site of dermis components. Therefore, according to the present invention, it is possible to provide a porous material as the artificial dermis for effectively promoting curing of wound.

Furthermore, since a porous material including the crosslinked material according to the present invention has uniform pores inside thereof and has a living body compatibility and property of gradually disassembling in a living body, the porous material carries a medicament(s) such as a growth factor (b-FGF, BMP), antibiotics. Therefore, it can be used for the porous material for a drug delivery system (DDS), which make its supply to a required portion such as an affected site. Furthermore, the porous material can be used variously as scaffolding materials for regenerative medicine and medical materials such as transplant materials in addition to artificial dermis.

The crosslinked material according to the present invention can be used as a bone regeneration base material. The crosslinked material may contain a bone morphogenetic factor and a growth factor. Examples of the bone morphogenetic factor may include bone morphogenetic protein (BMP) and recombinant human bone morphogenetic protein (rhBMP). Examples of the growth factor may include fibroblast growth factor (FGF), transformation growth factor beta (TGF-β), epithelium growth factor (EGF), insulin-like growth factor (IGF), platelet-derived growth factor (PDGF), endothelial cell growth factor (VEGF), neurotrophic factor (NGF) and at least one of them may be used.

The crosslinked material according to the present invention can be used as cartilage regeneration base materials for medical use and scaffold materials for cell culture. For example, the collagen/elastin porous material is homogeneous and has a preferable porous structure excellent in the invasiveness of cells. Therefore, cartilage-like tissue can be maintained. After the regeneration of the cartilage, since it is rapidly absorbed by a living body, it is applied to the injured surface of the cartilage, and the cartilage-like tissue can be effectively regenerated. Therefore, according to the present invention, it is possible to provide a cartilage regeneration base material that effectively promotes the regeneration of the cartilage.

The crosslinked material according to the present invention may contain a cartilage differentiation inducer and a growth factor that can be used as cartilage regeneration base materials for medical use and scaffold materials for cell culture. Examples of the cartilage differentiation inducer may include transformation growth factor beta (TGF-β), bone morphogenetic protein (BMP), recombinant human bone morphogenetic protein (rhBMP), Sox gene group (Sox9, Sox5 or Sox6) and at least one of them may be used.

The collagen/elastin crosslinked material of the present invention can be used as a base material for cosmetics. As the base material for cosmetics, it can be used for a face mask for cosmetics, wet sheet. In particular, when the collagen/elastin crosslinked material is a porous material, it can be impregnated with much skin lotion or essence, and the functionality of collagen and elastin can be expected. When the collagen/elastin crosslinked material is used as a face mask for cosmetics, it is possible to use a sheet-like porous material having a thickness of about 1 to 5 mm and having a shape corresponding to the shape of a face or a shape partially covering the face.

### Examples

Next, the description of the embodiment is described in detail with reference to Examples, but it is not construed that the present invention is limited to the following Examples. In the following Examples, "%" is based on the weight unless otherwise noted.

### [Example 1]

### (Cytotoxic Test of Fish-Derived Atelocollagen and Water Soluble Elastin)

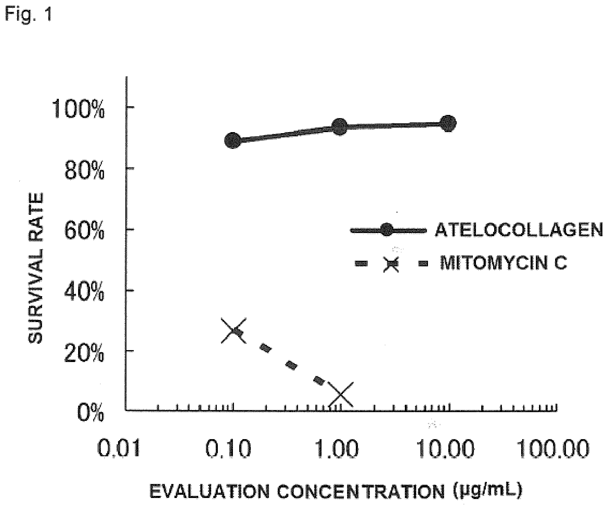

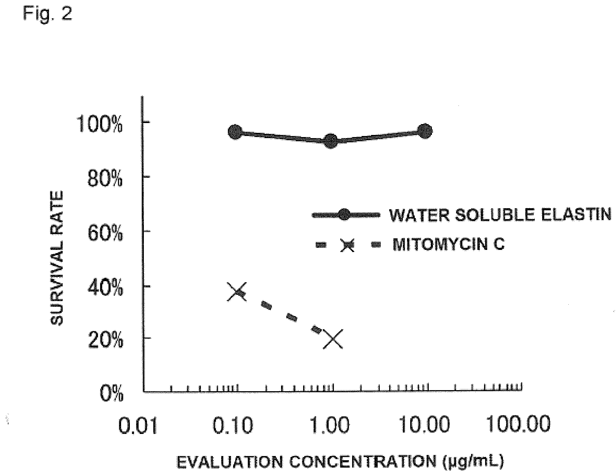

As samples, atelocollagen derived from the salmon skin and water soluble elastin derived from the salmon heart were used. For cells to be evaluated, human skin normal fibroblast that is a main component of the skin matrix was used. Cytotoxic test was carried out by a WST-1 assay. The WST-1 assay is a method well known to a person skilled in the art in which formazan produced when WST-1 is decomposed by the action of mitochondria in a cell is subjected to a colorimetric assay (measurement wavelength: 450 nm) to obtain an absorbance, and the number of living cells is measured by using the correlation between the absorbance and the number of living cells. The obtained results are shown in Figs. 1 and 2. In any samples, it was confirmed that the cell survival rate was high at the concentration of 0.1 to 10 µg/mL and the cell toxicity was not shown.

### [Example 2]

### (Preparation Example of Fish-Derived Collagen/Elastin Porous Material Using Crosslinking Agent)

A porous material was prepared by using the atelocollagen derived from the salmon skin and water soluble elastin derived from the salmon heart that were the same as those used in Example 1. More specifically, the atelocollagen derived from the salmon skin and water soluble elastin derived from the salmon heart were measured and taken out into a test tube so that the blending ratio (weight ratio) of collagen and elastin was 8:2. Deionized water was added to the mixed collagen/elastin powder, and stirred under refrigeration (3°C) to obtain a collagen/elastin mixed solution having a concentration of 5%. To the prepared collagen/elastin mixed solution, an aqueous solution of EDC (crosslinking agent: 1-Ethyl-3-(3-dimetylaminopropyl)-carbodiimide hydrochloride, Tokyo Chemical Industry Co., Ltd) was added under refrigeration (3°C) and mixed (EDC was added so that it became 20% (w/w) with respect to the total weight of the collagen and elastin). The collagen/elastin mixed solution to which EDC had been added was poured into a mold having an objective shape and lyophilized to obtain a collagen/elastin porous material. The obtained collagen/elastin porous material was washed with ion exchange water. After washing, the collagen/elastin porous material in a water retaining state was lyophilized to obtain a collagen/elastin porous material.

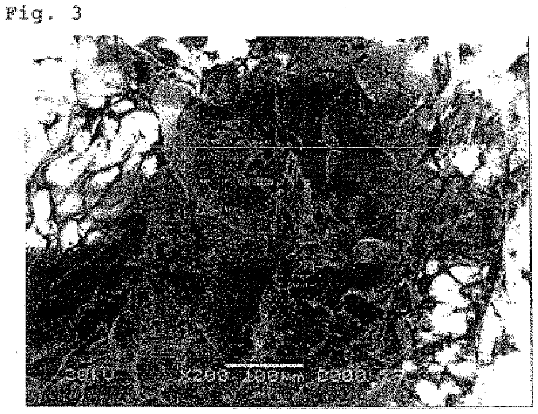

As a result of observing the structure of the obtained collagen/elastin porous material by a scanning electron microscope (manufactured by JEOL Ltd., JSM-6380LV, hereinafter, referred to as "SEM"), the sizes of the pores were 50 to 100 µm and the pore diameters were uniform (see Fig. 3).

### [Example 3]

### (Preparation Example of Fish-derived Collagen/Elastin Porous Material Using Heat Crosslinking)

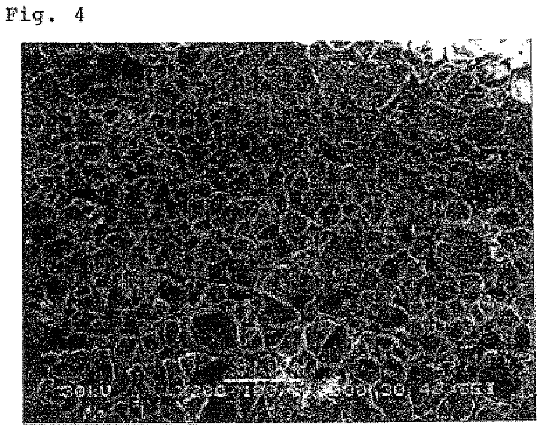

A porous material was prepared by using the atelocollagen derived from the salmon skin and water soluble elastin derived from the salmon heart that were the same as those used in Example 1. More specifically, the atelocollagen derived from the salmon skin and water soluble elastin derived from the salmon heart were measured and taken out into a test tube so that the blending ratio (weight ratio) of collagen and elastin was 7:3. Deionized water was added to the mixed collagen/elastin powder, and stirred under refrigeration (3°C) to obtain a collagen/elastin mixed solution having a concentration of 10%. The mixed solution was poured into a mold having an objective shape and lyophilized to obtain a lyophilized product. The lyophilized product was subjected to heat crosslinking under reduced pressure at 150°C for 24 hours to obtain a collagen/elastin porous material. The obtained porous material was put into water, and as a result, the shape was maintained for one week or more. As a result of observing the structure of the obtained collagen/elastin porous material by a scanning electron microscope (manufactured by JEOL Ltd., JSM-6380LV, hereinafter, referred to as "SEM"), the sizes of the pores are 50 to 100 µm and the pore diameters were uniform (see Fig. 4).

### [Example 4]

### (Change of Physical Property of Porous Material by Addition of Elastin to Collagen)

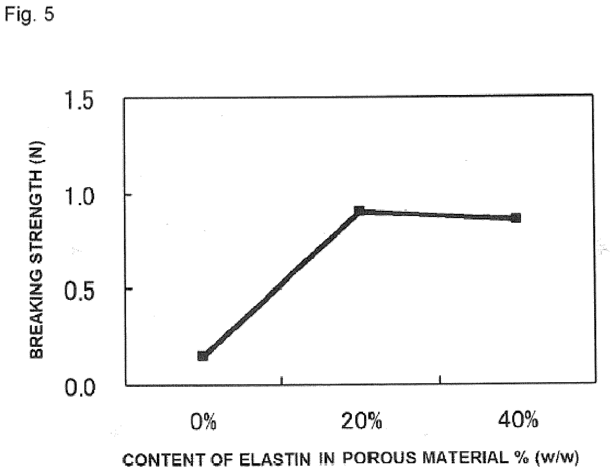

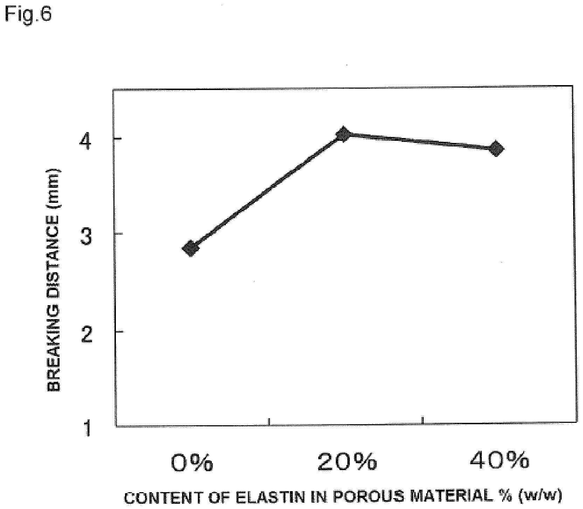

By using atelocollagen derived from the salmon skin and water soluble elastin derived from the salmon heart and by the same crosslinking method as in Example 2, a porous material containing 100% collagen, a porous material containing 20% (w/w) elastin to collagen, and a porous material containing 40% (w/w) elastin to collagen were prepared in the same size and the same shape (columnar shape having diameter of 12 mm and thickness of 6 mm) (the total concentration of collagen and elastin was made to be 5%.). These porous materials were left in water for 24 hours to obtain swollen-state gel. The mechanical strength of this gel was measured by using REOMETER (manufactured and sold by Sun Scientific Co., Ltd.). As a result, with the addition of elastin, as compared with the case of collagen alone (content of elastin: 0%), the breaking strength of the gel and breaking distance that is a value showing flexibility of the gel were increased (see Figs. 5 and 6). Furthermore, as a comparative control, commercially available artificial dermis using mammalian-derived collagen was measured in the same analysis conditions as mentioned above, and it was found that the strength was weak and the breaking distance was not measurable.

### [Example 5]

### (Evaluation of Fish-derived Collagen/Elastin Porous Material as Scaffold (Scaffolding Material for Cell Culture))

A fish-derived collagen/elastin porous material (diameter: 11 mm × thickness: 3 mm) prepared by the same method as in Example 2. For cells to be evaluated, WFB (immortalized fibroblast cell line of WKA rats) and human skin normal fibroblast were used. A porous material obtained by sterilizing a collagen/elastin porous material with ethylene oxide gas and degassing the material was placed in 12-well culture plate. Next, each sponge was impregnated with 1 mL each of a culture solution containing cells (cell concentration: 5 × 10⁵ cells/mL), and then 1 mL of the culture solution was poured thereto, which was cultured while a culture solution was replaced every three or four days. On Days 14 and 28, the survival checking and histological evaluation of cells were carried out. The survival of cells was checked by using the modified WST-1 assay. More specifically, for removing cells attached to the culture plate and measuring only cells taken by the porous material, the porous material was transferred to a new culture plate. A WST-1 solution - culture solution (1:10) in the amount of 2 to 3 mL was poured into a new culture plate, reacted for 4 hours, and the absorbance was measured by a plate reader. The histological evaluation was carried out by fixing a collagen/elastin porous material in formalin, which was embedded in a paraffin block, cut thinly, and subjected to hematoxylin-eosin staining.

Results of the modified WST-1 assay are shown in Table 1.

### [Table 1]

**TABLE 1**

| | CELL | ABSORBANCE (O.D.) | |
|---|---|---|---|
| CELLS TO BE EVALUATED | CONCENTRATION (cells/mL) | ON DAY 14 | ON DAY 28 |
| HUMAN SKIN NORMAL FIBROBLAST | 5×10⁵ | 0.78 | 0.81 |
| WFB | 5×10⁵ | 0.14 | 0.54 |

As shown in Table 1, in both cells, absorbance is apparently present as compared with blank (cell free), and the absorbance is increased as compared with the cells on Days 14 and 28, and therefore, survival and proliferation of the cells in the porous material were suggested. The cultured tissue was stained and observed under a microscope. As a result, in the human skin normal fibroblast and WFB, cells were observed to be attached to each other, and attached to the porous material. From these things, it was revealed that the fish-derived collagen/elastin porous material was able to be used as a scaffolding material for cell culture.

### [Example 6]

### (Implant Test of Fish-derived Collagen/Elastin Porous Material in Living Body (Rat Subcutis))

A fish-derived collagen/elastin porous material (diameter: 11 mm × thickness: 5 mm) prepared by the same method as in Example 2 was used. Wister rats (4-week old, about 200 g, female) were anesthetized with ether, and, in each rat, hair on the back was shaved with a hair-clipper. The skin of the back was sectioned with scissors, a pocket having a diameter of about 1 cm was formed under the subcutis (under the dartos), a porous material that had been sterilized with ethylene oxide gas was embedded, and then the wound was closed by suturing with nylon yarn. On Days 3, 7, 14, 21, 28, and 56 after operation, the rats were sacrificed deliberately, and the portion in which the porous material had been embedded was subjected to biopsy. The tissue was fixed in formalin, then it was made into a paraffin specimen. This was subjected to hematoxylin-eosin staining so as to evaluate living body compatibility.

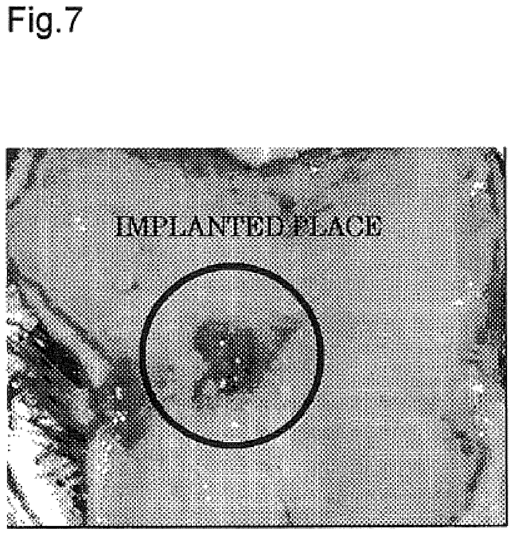

As a result of observation of the tissue into which a porous material had been implanted, in the early time of the implantation, infiltration of inflammatory cells, capillary blood vessels, and so-called granulation tissues of fibroblast and endothelial cells were observed in many places in the periphery of the porous material, then granulation tissue was gradually proliferated, new collagenous fibers (collagen) were produced, and completely cicatrized on Day 56. On the other hand, a porous material was destructed and absorbed from the periphery, and completely absorbed on Day 56 (see Fig. 7). This was substantially the same as the results of the animal experiment using artificial dermis using collagen, Pelnac (distributor; Johnson & Johnson K.K., manufacturer; Gunze Limited), which had been previously reported.

### [Example 7]

### (Transplant Test of Fish-Derived Collagen/Elastin Porous Material into Defective Wound Rat Skin)

A fish-derived collagen/elastin porous material (diameter 20 mm × thickness 5 mm) was prepared by the same method as in Example 2, and used as a transplant material in a sample group. In a control group, Terudermis that is a commercially available artificial dermis using collagen (collagen single layered type, manufactured by Terumo Corporation) was used as a transplant material. Wister rats were anesthetized with ether, and, in each rat, a defective wound having a diameter of about 15 mm was made on the back of the rat, transplant material was placed in the defective wound, the defective wound and the transplant material were completely covered with a dressing protective material, and the dressing protective material and skin stump were sutured with nylon yarn. In order to bring the transplant material and the bottom tissue into close contact with each other, they were slightly pressed with gauze, and further pressed and fixed to each other by using a stretchable band. Three groups, an OPEN WOUND group without using a transplant material, a sample group using a fish-derived collagen/elastin porous material as a transplant material, and a control group using Terudermis as a transplant material were formed, and five rats in each group were subjected to the same treatment.

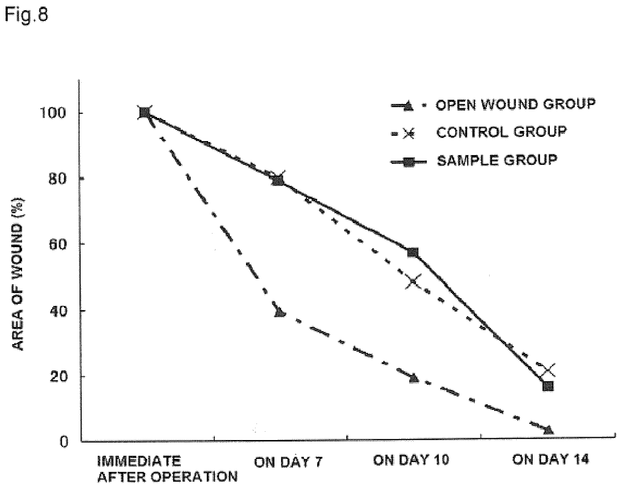

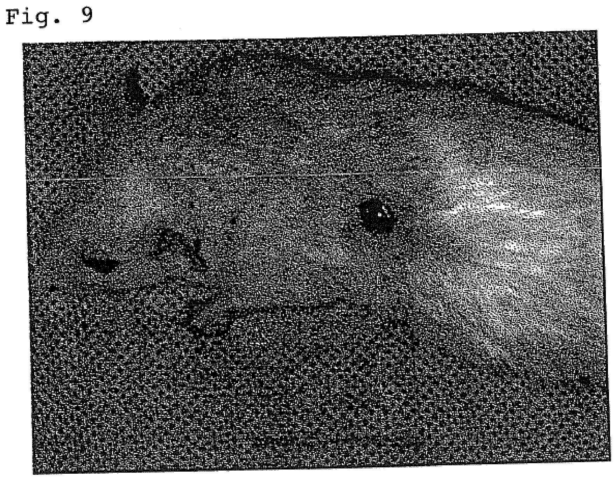

Findings of death, infection due to implantation of samples as a result of the test, were not observed in each group. According to the findings of macroscopic examination of the wound surface, in the sample group, no rapid contraction of the wound surface was observed after operation. On Day 7, the wound surface, most of which was granulated, of the OPEN WOUND group started to contract rapidly after operation, and on Day 14, the wound was almost cured. As a result of the comparison of sizes of wound surfaces over time, it was revealed that the wound surfaces of the OPEN WOUND group started to contract rapidly in the early time after operation, while the wound contraction in the sample group and the control group occurred relatively gradually (see Figs. 8 and 9). As a result of the histological examination of the wound surface (cured surface), in the sample group on Day 7 after operation, in the peripheral tissue, inflammatory cell infiltration, fibroblasts and endothelial cells were observed. On Day 14 after operation, bottom tissue was granulated and scar tissue, the regenerated epithelium stretched from the skin stump on the upper part thereof. The bottom portion of the tumor was rich in cell components and capillary blood vessels, and in the depth region, cell components were reduced and scar was maturing. The result was the same as the result of Terudermis that is a commercially available artificial dermis.

### [Example 8]

### (Preparation Example of DNA-added Collagen/Elastin Porous Material using Crosslinking Agent)

A porous material was prepared by using the atelocollagen derived from the salmon skin and water soluble elastin derived from the salmon heart that were the same as those used in Example 1. More specifically, the atelocollagen derived from the salmon skin and water soluble elastin derived from the salmon heart were measured and taken out into a test tube so that the blending ratio (weight ratio) of collagen and elastin was 8:2. Deionized water was added to the mixed collagen/elastin powder, and stirred under refrigeration (3°C) to obtain a collagen/elastin mixed solution having a concentration of 5%. Deionized water was added to DNA derived from salmon milt, and the solution was stirred under refrigeration (3°C) to obtain a DNA solution having a concentration of 5%.

To the previously prepared collagen/elastin mixed solution having a concentration of 5%, the DNA solution having a concentration of 5% was added so that the weight ratio became 8:2, the mixture was stirred under refrigeration (3°C) to obtain a DNA-added collagen/elastin mixed solution. To the DNA-added collagen/elastin mixed solution, an aqueous solution of EDC (crosslinking agent: 1-Ethyl-3-(3-dimetylaminopropyl)-carbodiimide hydrochloride, Tokyo Chemical Industry Co., Ltd.) was added and mixed (added so that EDC became 20% (w/w) with respect to the total weight of collagen/elastin and DNA). The DNA-added collagen/elastin mixed solution to which EDC had been added was poured into a mold having an objective shape and lyophilized to obtain a DNA-added collagen/elastin porous material. The obtained DNA-added collagen/elastin porous material was washed with ion exchange water. After washing, a DNA-added collagen/elastin porous material in a water retaining state was lyophilized to obtain a DNA-added collagen/elastin porous material.

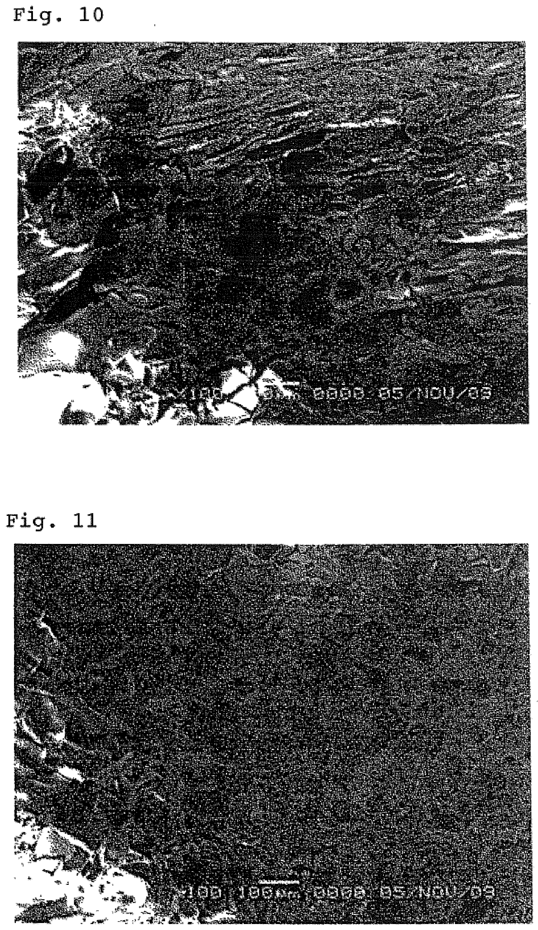

As a result of observing the structure of the obtained DNA-added collagen/elastin porous material by a scanning electron microscope (manufactured by JEOL Ltd., JSM-6380LV, hereinafter, referred to as "SEM"), the sizes of the pores were about 100 µm and the pore diameters were uniform (see Fig. 10).

### [Example 9]

### (Preparation Example of Protamine-added Collagen/Elastin Porous Material using Crosslinking Agent)

A porous material was prepared by using the atelocollagen derived from the salmon skin and water soluble elastin derived from the salmon heart that were the same as those used in Example 1. More specifically, the atelocollagen derived from the salmon skin and water soluble elastin derived from the salmon heart were measured and taken out into a test tube so that the blending ratio (weight ratio) of collagen and elastin was 8:2. Deionized water was added to the mixed collagen/elastin powder, and stirred under refrigeration (3°C) to obtain a collagen/elastin mixed solution having a concentration of 5%. Deionized water was added to protamine derived from salmon milt, and the solution was stirred under refrigeration (3°C) to obtain a protamine solution having a concentration of 5%. To the previously prepared collagen/elastin mixed solution having a concentration of 5%, the protamine solution having a concentration of 5% was added so that the weight ratio became 8:2, the mixture was stirred under refrigeration (3°C) to obtain a protamine-added collagen/elastin mixed solution. To the protamine-added collagen/elastin mixed solution, an aqueous solution of EDC (crosslinking agent: 1-Ethyl-3-(3-dimetylaminopropyl)-carbodiimide hydrochloride, Tokyo Chemical Industry Co., Ltd.) was added and mixed (added so that EDC became 20% (w/w) with respect to the total weight of collagen/elastin and protamine). The protamine-added collagen/elastin mixed solution to which EDC had been added was poured into a mold having an objective shape and lyophilized to obtain a protamine-added collagen/elastin porous material. The obtained protamine-added collagen/elastin porous material was washed with ion exchange water. After washing, a protamine-added collagen/elastin porous material in a water retaining state was lyophilized to obtain a protamine-added collagen/elastin porous material.

As a result of observing the structure of the obtained protamine-added collagen/elastin porous material by a scanning electron microscope (manufactured by JEOL Ltd., JSM-6380LV, hereinafter, referred to as "SEM"), the sizes of the pores were about 100 µm and the pore diameters were uniform (see Fig. 11).

### [Example 10]

### (Preparation Example of Hydroxyapatite-added Collagen/Elastin Porous Material Using Crosslinking Agent)

A porous material was prepared by using the atelocollagen derived from the salmon skin and water soluble elastin derived from the salmon heart that were the same as those used in Example 1. More specifically, the atelocollagen derived from the salmon skin and water soluble elastin derived from the salmon heart were measured and taken out into a test tube so that the blending ratio (weight ratio) of collagen and elastin was 8:2. Deionized water was added to the mixed collagen/elastin powder, and stirred under refrigeration (3°C) to obtain a collagen/elastin mixed solution having a concentration of 5%. Hydroxyapatite (hereinafter, referred to as "HA") was added to the prepared collagen/elastin mixed solution so that the weight ratio of the total weight amount of collagen and elastin to the weight of HA became 8:2, and kneaded under refrigeration (3°C). To the HA-added collagen/elastin mixed solution, an aqueous solution of EDC (crosslinking agent: 1-Ethyl-3-(3-dimetylaminopropyl)-carbodiimide hydrochloride, Tokyo Chemical Industry Co., Ltd.) was added and mixed (added so that EDC became 20% (w/w) with respect to the total weight of collagen and elastin). The HA-added collagen/elastin mixed solution to which EDC had been added was poured into a mold having an objective shape and lyophilized to obtain an HA-added collagen/elastin porous material. The obtained HA-added collagen/elastin porous material was washed with ion exchange water. After washing, an HA-added collagen/elastin porous material in a water retaining state was lyophilized to obtain an HA-added collagen/elastin porous material.

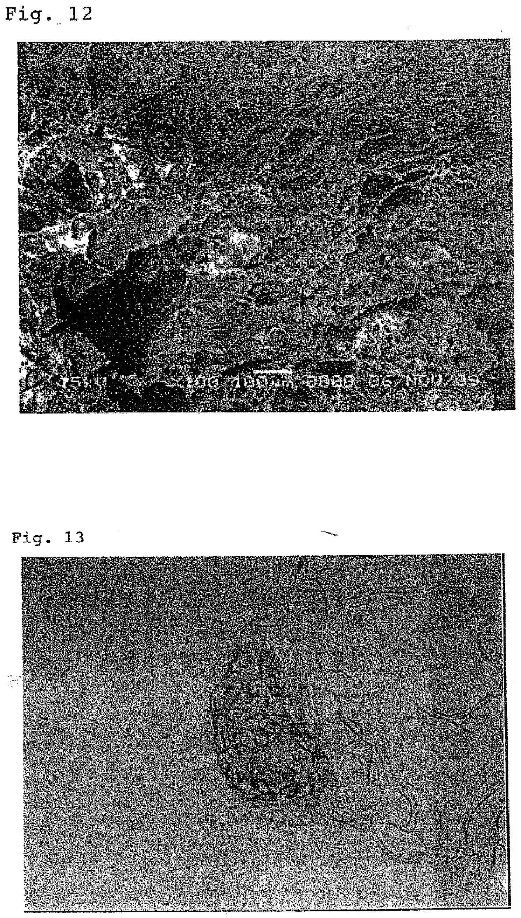

As a result of observing the structure of the obtained HA-added collagen/elastin porous material by a scanning electron microscope (manufactured by JEOL Ltd., JSM-6380LV, hereinafter, referred to as "SEM"), the sizes of the pores were about 100 µm (see Fig. 12).

### [Example 11]

### (Use of Collagen/Elastin Porous Material in Field of Cartilage Regeneration)

A fish-derived collagen/elastin porous material (diameter: 11 mm × thickness: 5 mm) that had been prepared by the same method as in Example 2. The collagen/elastin porous material that had been subjected to degassing in the culture solution was placed in 10 cm-culture dish. Next, the cultured chondrocytes were recovered from the culture flask and the number of cells was counted, 5 × 10⁶ cells were suspended again in 500 µL of culture solution to obtain a cell culture solution. The collagen/elastin porous material was impregnated with the cell culture solution, and started to be cultured in an incubator. Furthermore, when 12 hours had passed, 10 mL of culture solution was poured into the culture dish. The culture solution was replaced every three or four days. On Day 18 after culture, tissue was recovered and fixed in formalin, embedded in a paraffin block and cut thinly, and thus tissue section was formed. The tissue section was subjected to hematoxylin-eosin staining, alcian blue staining, toluidine blue staining, and immunostaining with collagen II antibody.

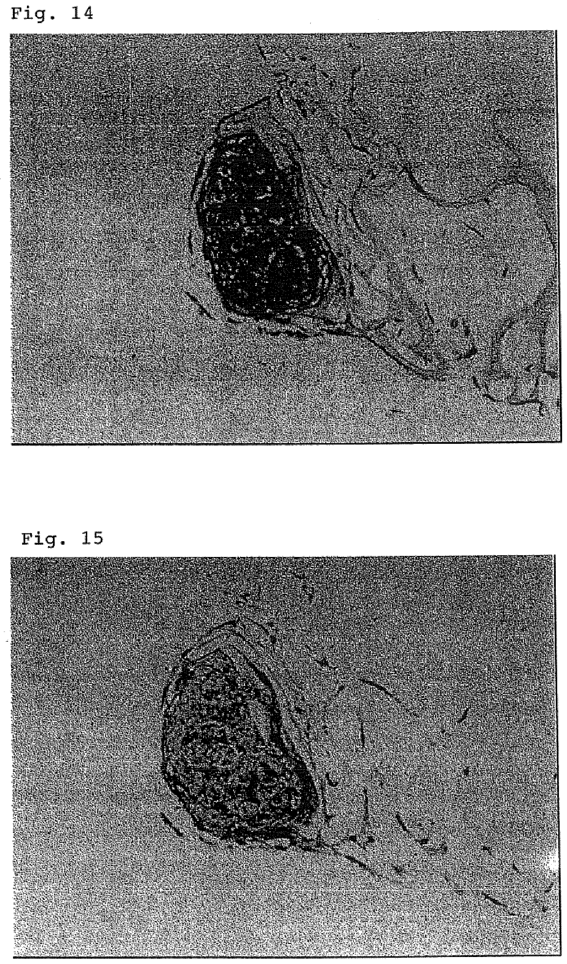

Since a substrate in the periphery of cells is deeply stained by alcian blue staining (Fig. 13) and toluidine blue staining (Fig. 14), it was thought that the substrate was a cartilage substrate containing a large amount of acidic mucopolysaccharides such as chondroitin sulfate and hyaluronic acid. Meanwhile, in the toluidine blue staining, characteristic metachromasy to a chondrocyte was observed. Furthermore, in the immunostaining with collagen II antibody which stains collagen II contained characteristically in the cartilage substrate, the substrate was stained deeply, and it was confirmed that a cartilage substrate was produced in the periphery of cells (Fig. 15). From the above-mentioned results, it was suggested that the collagen/elastin porous material was used for a chondrocyte culture base material (scaffold) or a cartilage regeneration base material.

### [Example 12]

### (Preparation of Film-like Fish-derived Collagen/Elastin Complex)

A membrane was prepared by using the atelocollagen derived from the salmon skin and the water soluble elastin derived from the salmon heart that were the same as those used in Example 1. More specifically, the atelocollagen and water soluble elastin were measured and taken out into a test tube so that the blending ratio (weight ratio) of collagen and elastin was 8:2. Deionized water was added to the mixed collagen/elastin powder to obtain a collagen/elastin mixed solution having a concentration of 20%. To the prepared collagen/elastin mixed solution, an aqueous solution of EDC (crosslinking agent: 1-Ethyl-3-(3-dimetylaminopropyl)-carbodiimide hydrochloride, Tokyo Chemical Industry Co., Ltd.) was added and mixed (added so that EDC became 0.5% (w/w) with respect to the total weight of the collagen and elastin). The mixed solution to which EDC had been added was applied onto a sheet made of polypropylene, and this is allowed to stand still in a desiccator containing a drying agent, dried and fixed (the above-mentioned operation was carried out at 5°C or less.). After the solution was dried and fixed, a film-like collagen/elastin complex was heat treated under reduced pressure at 180°C for one hour.

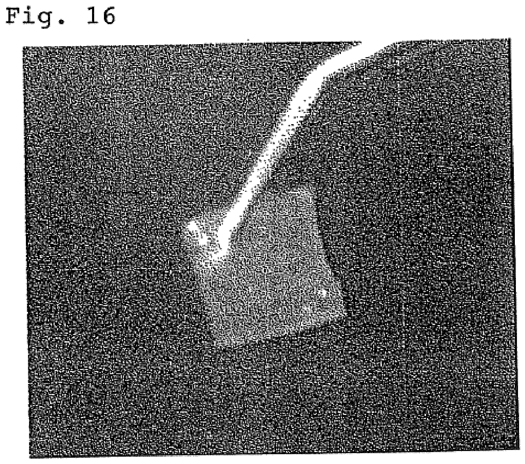

When the obtained film-like collagen/elastin complex was immersed in water, it maintained the shape for one week or more (Fig. 16).

### [Example 13]

### (Use of Collagen/Elastin Complex as Cosmetics Base Material)

In subjects (four male subjects of ages: 20 to 39), left forearm was washed well with soap and rinsed with water, followed by drying by wiping. After washing for 30 minutes, three sites at the inner side of the left forearm to which probe of measurement device are placed were marked. The viscoelasticity in the marked sites was measured by using a skin viscoelasticity measurement device, Cutometer MPA580 (Courage + Khazaka Electronic GmbH, measurement mode: 1) (before applying: time 0). Time 0, after measuring, to one portion of the marks of three points of each subject, a sheet-like collagen/elastin porous material impregnated with commercially available skin lotion was attached and left for 15 min. Note here that the sheet-like collagen/elastin porous material was prepared as in Example 2. After 15 min have passed, the sheet-like collagen/elastin porous material impregnated with skin lotion (referred to as "CES + skin lotion applying") was peeled off and at the same time, skin lotion was applied on another one marked portion. Similarly, to total four subjects, a not-applied portion, a skin lotion-applied portion, a CES + skin lotion applied portion were formed. Two hours after the applying, the viscoelasticity of the marked sites was measured similar to the above.

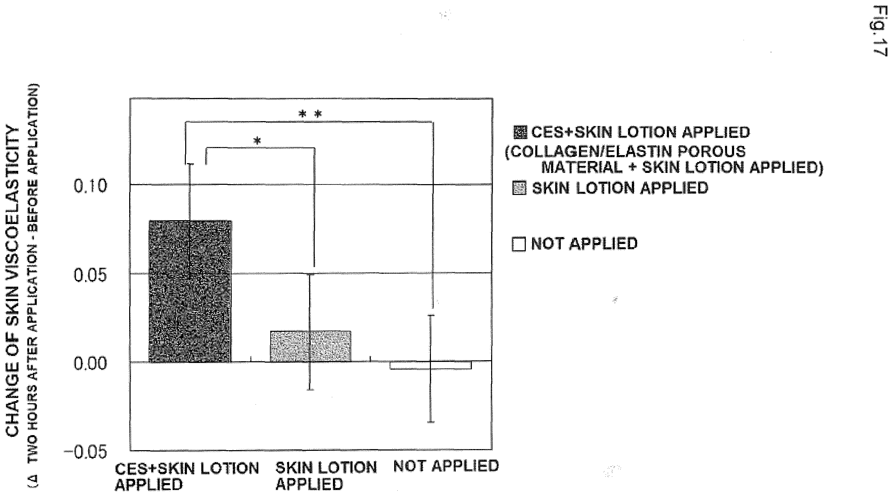

When difference of the viscoelasticity values (R7 values) of each portion between before and after the application was compared, in a CES + skin lotion applied portion, skin viscoelasticity was significantly increased as compared with the not-applied portion (Fig. 17). Furthermore, in the macroscopic finding of the CES + skin lotion applied portion, no abnormality was observed. As mentioned above, since the collagen/elastin porous material increased the effect of cosmetics, it was able to be used as the cosmetics base material.

### [Example 14]

### (Preparation (2) of Film-Like Fish-Derived Collagen/Elastin Complex)

A membrane was prepared by using the atelocollagen derived from the salmon skin and the water soluble elastin derived from the salmon heart that were the same as those used in Example 1. More specifically, the atelocollagen and water soluble elastin were measured and taken out into a test tube so that the blending ratio (weight ratio) of collagen and elastin was 8:2. Deionized water was added to the mixed collagen/elastin powder to obtain a collagen/elastin mixed solution having a concentration of 20%. The mixed solution was applied onto a sheet made of polypropylene, and this was allowed to stand still in a desiccator containing a drying agent, dried and fixed (the above-mentioned operation was carried out at 5°C or less.). After the solution was dried and fixed, a film-like collagen/elastin complex was heat treated under reduced pressure at 180°C for one hour to prepare a film-like collagen/elastin crosslinked material.

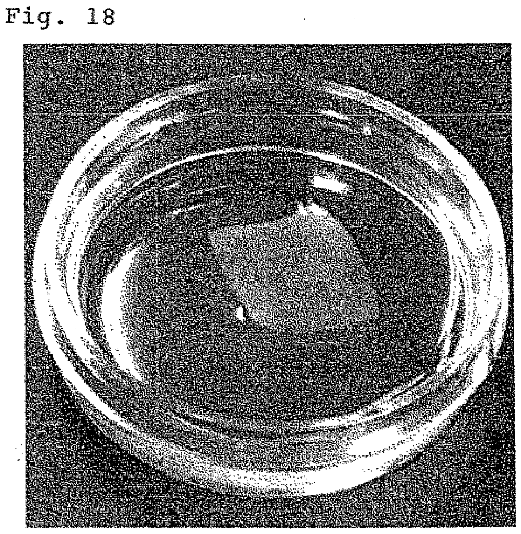

When the obtained film-like collagen/elastin complex was immersed in water, it maintained the shape in water for one week or more (Fig. 18).

## Claims

1. A porous crosslinked material, wherein the porous crosslinked material is obtained by a production method comprising the steps of:
- preparing a solution in which a fish-derived collagen and a fish-derived elastin having a weight-average molecular weight of 10,000 to 100,000 are dissolved at a temperature of 0 to 10°C and pH ranging from 3 to 6, whereby coacervation of the elastin is not generated;
- obtaining a dried product from the solution; and
- forming a crosslinked material by crosslinking the dried product.

2. The porous crosslinked material according to claim 1, wherein the dried product is obtained by lyophilization.

3. The porous crosslinked material according to any one of claims 1 to 2, wherein the solution further comprises at least one of DNA and protamine.

4. The porous crosslinked material according to any one of claims 1 to 3, further comprising calcium phosphate.

5. A method of producing a porous crosslinked material, the method comprising the steps of:
- preparing a solution in which a fish-derived collagen and a fish-derived elastin having a weight-average molecular weight of 10,000 to 100,000 are dissolved at a temperature of 0 to 10°C and pH ranging from 3 to 6, whereby coacervation of the elastin is not generated;
- obtaining a dried product from the solution; and
- forming a crosslinked material by crosslinking the dried product.

6. The method of producing a porous crosslinked material according to claim 5, wherein the dried product is obtained by lyophilization.

7. The method of producing a porous crosslinked material according to any one of claims 5 to 6, wherein the solution further comprises at least one of DNA and protamine.

8. The method of producing a porous crosslinked material according to any one of claims 5 to 7, wherein the crosslinked material further comprises calcium phosphate.

9. A medical material comprising a porous crosslinked material according to any one of claims 1 to 4.

10. The medical material according to claim 9, wherein the medical material is selected from artificial dermis, cartilage regeneration base material, bone regeneration base material and a bone regeneration base material supporting a bone morphogenetic factor or a bone growth factor.

11. A scaffolding material for cell culture, comprising a crosslinked porous material according to any one of claims 1 to 4.

12. The scaffolding material for cell culture according to claim 11, wherein the scaffolding material is suitable for culturing a cartilage cell or for culturing a bone cell.

13. A cosmetics base material comprising a crosslinked porous material according to any one of claims 1 to 4.

## Patentansprüche

1. Poröses vernetztes Material, wobei das poröse vernetzte Material erhalten wird durch ein Herstellungsverfahren, umfassend die Schritte von:
- Herstellen einer Lösung, in der ein von Fisch abgeleitetes Collagen und ein von Fisch abgeleitetes Elastin mit einem gewichtsmittleren Molekulargewicht von 10.000 bis 100.000 bei einer Temperatur von 0 bis 10°C und einem pH-Wert in einem Bereich von 3 bis 6 gelöst werden, wobei keine Koazervierung des Elastins stattfindet;
- Erhalten eines getrockneten Produkts aus der Lösung; und
- Bilden eines vernetzten Materials durch Vernetzen des getrockneten Produkts.

2. Poröses vernetztes Material gemäß Anspruch 1, wobei das getrocknete Produkt durch Lyophilisierung erhalten wird.

3. Poröses vernetztes Material gemäß einem der Ansprüche 1 bis 2, wobei die Lösung ferner mindestens eines von DNA und Protamin umfasst.

4. Poröses vernetztes Material gemäß einem der Ansprüche 1 bis 3, ferner umfassend Calciumphosphat.

5. Verfahren zum Herstellen eines porösen vernetzten Materials, wobei das Verfahren die folgenden Schritte umfasst:
- Herstellen einer Lösung, in der ein von Fisch abgeleitetes Collagen und ein von Fisch abgeleitetes Elastin mit einem gewichtsmittleren Molekulargewicht von 10.000 bis 100.000 bei einer Temperatur von 0 bis 10°C und einem pH-Wert in einem Bereich von 3 bis 6 gelöst werden, wobei keine Koazervierung des Elastins stattfindet;
- Erhalten eines getrockneten Produkts aus der Lösung; und
- Bilden eines vernetzten Materials durch Vernetzen des getrockneten Produkts.

6. Verfahren zum Herstellen eines porösen vernetzten Materials gemäß Anspruch 5, wobei das getrocknete Produkt durch Lyophilisierung erhalten wird.

7. Verfahren zum Herstellen eines porösen vernetzten Materials gemäß einem der Ansprüche 5 bis 6, wobei die Lösung ferner mindestens eines von DNA und Protamin umfasst.

8. Verfahren zum Herstellen eines porösen vernetzten Materials gemäß einem der Ansprüche 5 bis 7, wobei das vernetzte Material ferner Calciumphosphat umfasst.

9. Medizinisches Material, umfassend ein poröses vernetztes Material gemäß einem der Ansprüche 1 bis 4.

10. Medizinisches Material gemäß Anspruch 9, wobei das medizinische Material aus künstlicher Haut, Knorpelregenerationsbasismaterial, Knochenregenerationsbasis-material und einem Knochenregenerationsbasismaterial, das einen knochenmorphogenen Faktor oder einen Knochenwachstumsfaktor trägt, ausgewählt ist.

11. Gerüstmaterial für Zellkultur, umfassend ein vernetztes poröses Material gemäß einem der Ansprüche 1 bis 4.

12. Gerüstmaterial für Zellkultur gemäß Anspruch 11, wobei das Gerüstmaterial zum Kultivieren einer Knorpelzelle oder zum Kultivieren einer Knochenzelle geeignet ist.

13. Kosmetika-Basismaterial, umfassend ein vernetztes poröses Material gemäß einem der Ansprüche 1 bis 4.

## Revendications

1. Matériau réticulé poreux, le matériau réticulé poreux étant obtenu via un procédé de production comprenant les étapes dans lesquelles :
- on prépare une solution dans laquelle on dissout un collagène qui dérive d'un poisson et une élastine qui dérive d'un poisson possédant un poids moléculaire moyen en poids de 10.000 à 100.000 à une température de 0 à 10 °C et à un pH qui se situe dans la plage de 3 à 6, préparation qui ne donne pas lieu à une coacervation de l'élastine ;
- on obtient un produit séché à partir de la solution ; et
- on forme un matériau réticulé par réticulation du produit séché.

2. Matériau réticulé poreux selon la revendication 1, dans lequel on obtient le produit séché par lyophilisation.

3. Matériau réticulé poreux selon l'une quelconque des revendications 1 à 2, dans lequel la solution comprend en outre au moins un élément choisi parmi de l'ADN et de la protamine.

4. Matériau réticulé poreux selon l'une quelconque des revendications 1 à 3, comprenant en outre du phosphate de calcium.

5. Procédé de production d'un matériau réticulé poreux, le procédé comprenant les étapes dans lesquelles :
- on prépare une solution dans laquelle on dissout un collagène qui dérive d'un poisson et une élastine qui dérive d'un poisson possédant un poids moléculaire moyen en poids de 10.000 à 100.000 à une température de 0 à 10 °C et à un pH qui se situe dans la plage de 3 à 6, préparation qui ne donne pas lieu à une coacervation de l'élastine ;
- on obtient un produit séché à partir de la solution ; et
- on forme un matériau réticulé par réticulation du produit séché.

6. Procédé de production d'un matériau réticulé poreux selon la revendication 5, dans lequel on obtient le produit séché par lyophilisation.

7. Procédé de production d'un matériau réticulé poreux selon l'une quelconque des revendications 5 à 6, dans lequel la solution comprend en outre au moins un élément choisi parmi de l'ADN et de la protamine.

8. Procédé de production d'un matériau réticulé poreux selon l'une quelconque des revendications 5 à 7, dans lequel le matériau réticulé comprend en outre du phosphate de calcium.

9. Matériau médical comprenant un matériau réticulé poreux selon l'une quelconque des revendications 1 à 4.

10. Matériau médical selon la revendication 9, dans lequel le matériau médical est choisi parmi du derme artificiel, une matière de base pour la régénération du cartilage, une matière de base pour la régénération osseuse et une matière de base pour la régénération osseuse qui supporte un facteur morphogénétique osseux ou un facteur de croissance osseuse.

11. Matériau procurant un soutien pour une culture cellulaire, comprenant un matériau poreux réticulé selon l'une quelconque des revendications 1 à 4.

12. Matériau procurant un soutien pour une culture cellulaire selon la revendication 11, dans lequel le matériau procurant un soutien est approprié pour la mise en culture d'une cellule de cartilage ou pour la mise en culture d'une cellule osseuse.

13. Matériau cosmétique de base comprenant un matériau poreux réticulé selon l'une quelconque des revendications 1 à 4.
